(19) Europäisches Patentamt
European Patent Office
Office européen des brevets

(11) **EP 1 722 837 B1**

(12) **EUROPEAN PATENT SPECIFICATION**

(45) Date of publication and mention of the grant of the patent:
**30.04.2008 Bulletin 2008/18**

(21) Application number: **05708587.0**

(22) Date of filing: **03.03.2005**

(51) Int Cl.:
**A61M 1/30** (2006.01)

(86) International application number:
**PCT/IB2005/000468**

(87) International publication number:
**WO 2005/092408 (06.10.2005 Gazette 2005/40)**

(54) **SINGLE NEEDLE DEVICE FOR EXTRACORPOREAL TREATMENT OF BLOOD**

EINZELNADELVORRICHTUNG FÜR DIE EXTRAKORPORALE BEHANDLUNG VON BLUT

DISPOSITIF A AIGUILLE UNIQUE POUR LE TRAITEMENT EXTRACORPOREL DU SANG

(84) Designated Contracting States:
**AT BE BG CH CY CZ DE DK EE ES FI FR GB GR HU IE IS IT LI LT LU MC NL PL PT RO SE SI SK TR**

(30) Priority: **08.03.2004 FR 0402381**

(43) Date of publication of application:
**22.11.2006 Bulletin 2006/47**

(73) Proprietor: **Gambro Lundia AB**
**22010 Lund (SE)**

(72) Inventors:
• **CHEVALLET, Jacques**
  **F-69360 Serezin du Rhone (FR)**
• **ROMARIE, Jean-Louis**
  **69150 Decines Charpieu (FR)**
• **FRUGIER, Alain**
  **F-38230 Tignieu (FR)**

(74) Representative: **Villanova, Massimo et al**
**Gambro Intellectual Property Department,**
**P.O. Box 98**
**41037 Mirandola (MO) (IT)**

(56) References cited:
**EP-A- 1 184 046        WO-A-01/08557**
**FR-A- 2 672 217**

EP 1 722 837 B1

**Description**

**Technical field of the Invention:**

The present invention relates to a device for extracorporeal treatment of blood intended to be connected to a patient by means of a single needle, this device being for example an artificial kidney or a plasmapheresis device.

**Prior Art:**

With such devices, it is known to carry out the connection of the extracorporeal blood circuit to the patient by two modes of operation: the "double-needle" mode and the "single needle" mode.

In the double-needle mode, two needles are used: a first for the patient's arterial access, that is to say for taking the blood from the patient, and a second needle for the patient's blood access, that is to say for returning the blood into the patient. The blood thus flows in a circuit through which it passes entirely in one direction: first needle, arterial line, then blood compartment of the filter, then venous line, and finally second needle. This mode of operation makes it possible to take the blood and give it back simultaneously. However, this mode makes it necessary to insert two needles into the patient, for example in the patient's fistula. This double-needle mode is used very widely to obtain good efficiency but it has its limitations, in particular owing to the patient. This is because with successive treatments, at a rate of three sessions per week in the case of a chronic treatment, the patient's fistula may suffer damage and no longer permit advantageous insertion. A small fistula may prevent access for both needles, or may then entail insertion of two needles so close to each other that an excessive recirculation phenomenon would reduce the quality of the treatment.

By necessity and/or in order to avoid increasing the fragility of the patient's vascular access, therefore, the "single needle" mode may be opted for.

In the single-needle mode, a single needle is inserted into the patient, for example into his or her fistula. There are therefore two separate and alternating phases of operation.

The first phase is referred to as the arterial phase, during which the blood is taken from the patient via the single needle into the extracorporeal circuit, and the second phase is referred to as the venous phase, during which the blood is passed from the extracorporeal blood circuit back to the patient through the single needle. Owing to the use of a single needle, the flow of the blood throughout the extracorporeal circuit, including the needle, is not continuous and a certain volume of blood (referred to as the "stroke volume") has to be stored in the extracorporeal circuit during each cycle of operation. Problems therefore arise concerning the quality and quantity of blood treatment in relation to the session time.

A plurality of single-needle circuits have been proposed.

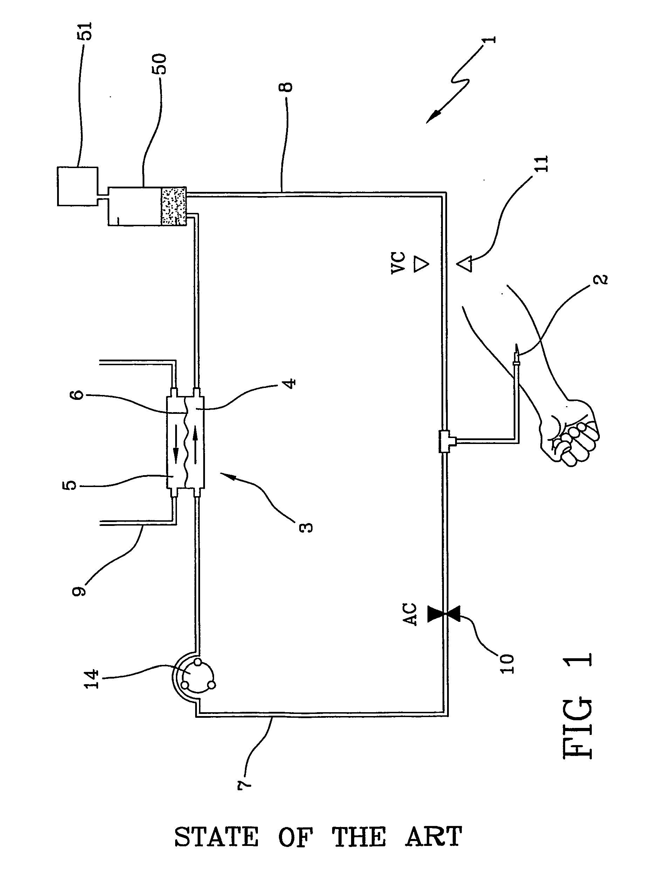

A first known circuit is schematically represented in Figure 1. The extracorporeal circuit 1 has two clamps, AC (arterial clamp 10) and VC (venous clamp 11), an arterial pump 14, and an expansion chamber 50 with a fixed volume to store the blood being treated. In an arterial phase, the patient's blood flows through the arterial line 7 and enters the treatment unit 3 then the venous line 8. Since the blood cannot be re-injected because the clamp 11 (VC) is closed, it is stored in the expansion chamber 50 in fluid communication with the venous line 8.

The capacity of the expansion chamber 50 is constant in volume, it contains only air before operation, and the volume of blood stored during operation increases and decreases alternately. The pressure in the chamber will be such that the product Volume x Pressure of blood remains constant. The greater the fluctuation in volume is, therefore, the greater will be that of the pressure, which is not desirable. It is therefore known to add a "dead volume" to the chamber, that is to say a volume of air added to that of the chamber by a second chamber 51 in air communication with the first chamber 50, in order to minimize the variations in volume and pressure inside the chamber 50.

Although the circuit is indeed simple, the blood flow rate in the dialyzer (or treatment unit) is not continuous, the arterial pump 14 does not operate continuously and the venous phase is not optimized because the flow rate in the needle is dictated by the pressure in the chamber. The problem of haemocompatibility furthermore arises, all the more so because of the expansion chamber 50 in which the surface of the air/blood interface entails coagulation problems.

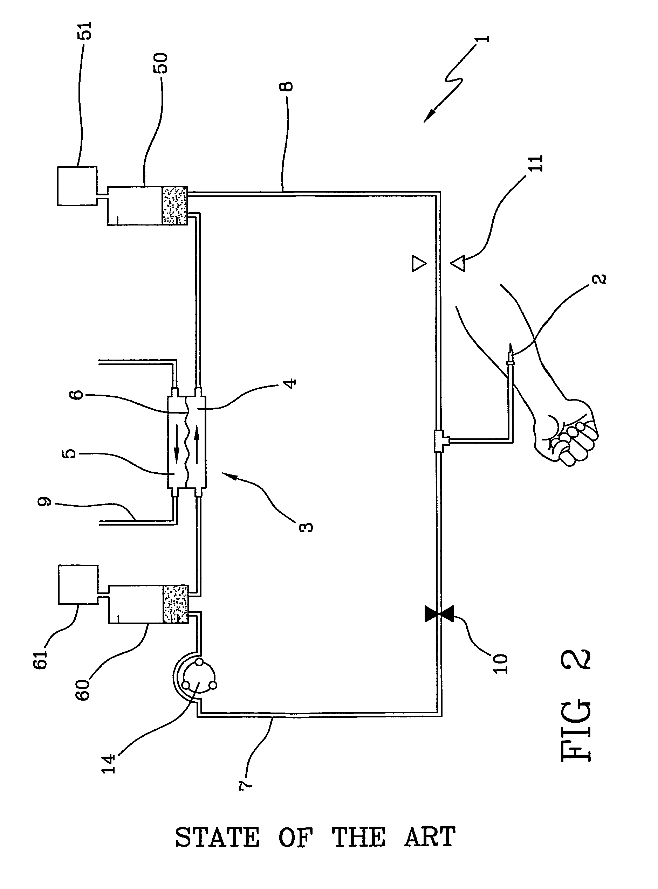

A second known circuit, proposed in Figure 2, is an improvement of the previous circuit. In order to obtain a continuous flow rate of blood in the dialyzer 3, the extracorporeal circuit which is schematically represented includes a second expansion chamber 60 connected to a second dead volume 61, for storing the blood. This allows the blood to pass continuously through the dialyser and increases the quality of dialysis treatment. It is, however, necessary for the volume in each of the two expansion chambers to be regulated correctly, so that it is identical and equal to half of the stroke volume: this fairly intricate operation is carried out by an operator. Other drawbacks remain: the fluctuating pressure of the blood, the nonoptimized venous phase, the problem of haemocompatibility and coagulation of the blood.

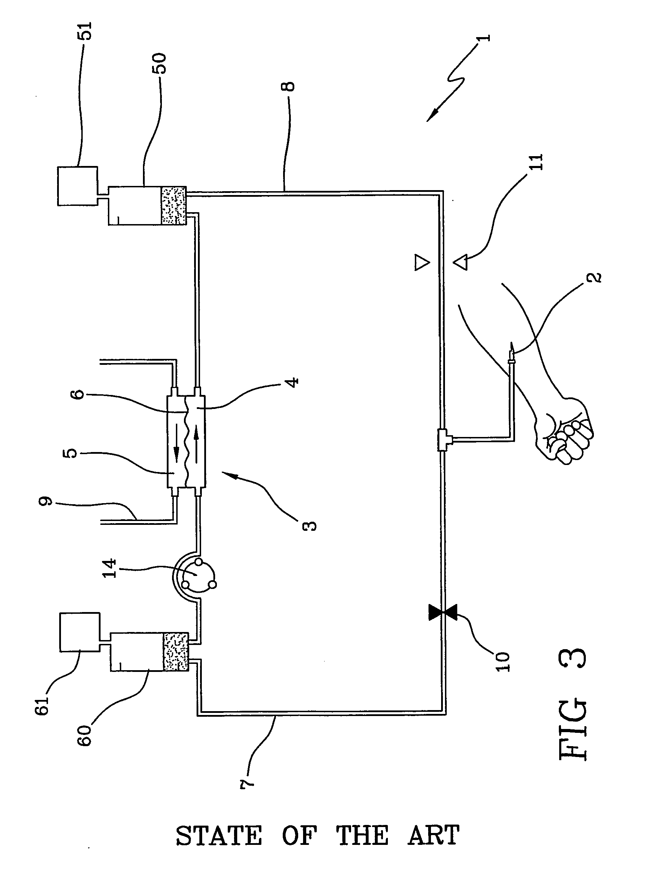

A third circuit is known and, in Figure 3, represents an improvement of the circuit in Figure 2. The difference from the second circuit is the position of the arterial pump 14, which is connected downstream of the expansion chamber 60 placed on the arterial line 7, delivering a constant flow rate in the dialyser 3. In general, the other drawbacks remain: the intake and delivery pressures vary during the arterial and venous phases, the fluctuating pressure of the blood, the problem of haemocompatibility and coagulation of the blood.

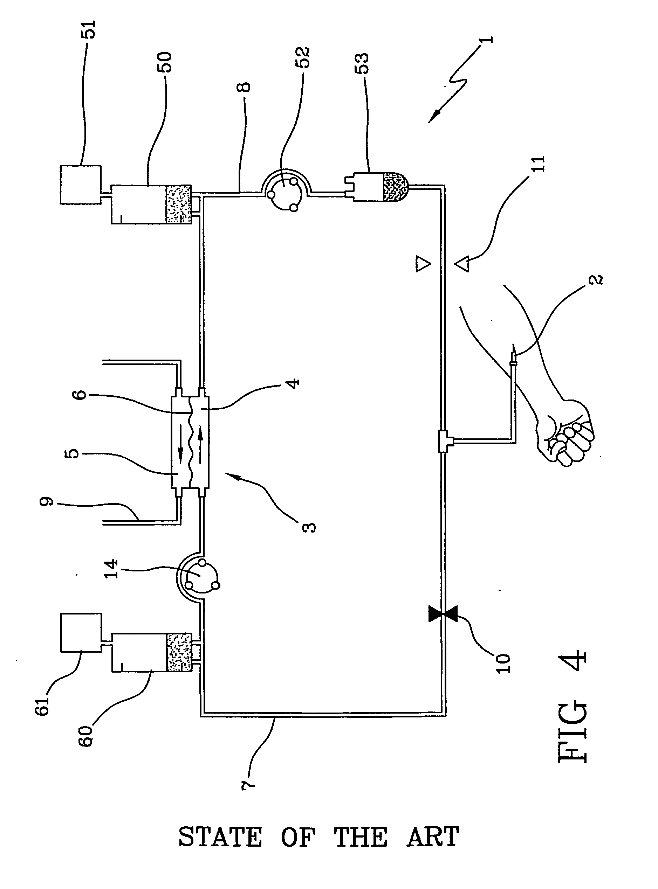

Next, with reference to Figure 4, a second pump 52 has been added downstream of the expansion chamber 50 placed on the venous line, and a bubble trap 53 has been added downstream of the second pump 52 but upstream of the venous clamp 11. This has made it possible to optimize the venous phase, because the flow rate is no longer dictated by the pressure of the expansion chamber 50. The haemocompatibility and the undesired coagulation remained problems, however, besides a circuit which has been made more complex.

Moreover, another problem to be dealt with is the regulation and control of such a device. In fact, an operator has to intervene for priming the circuit and regulating the expansion chambers 50 and 60. This requires the constant presence of staff with the patient, and precise and skilful intervention by these staff. There has been a desire to auto-regulate the operation of such machines.

A circuit 1 with a single needle 2, disclosed in Patent FR 2 584 906, is known and is illustrated in Figure 5. It includes an arterial pump 14 and two expansion chambers, arterial 50 and venous 60, for storing the blood. In order to auto-regulate the operation of the device, a high limit detector 54 (respectively a low limit detector 55) are connected onto the venous expansion chamber 50 for detecting a maximum (respectively minimum) quantity of blood being treated. The detected signals are used for control of the arterial and venous phases by a calculation and control unit 15. The limit detector includes a pressure value detector for the volume of air contained in each reservoir. The control procedure nevertheless remains intricate, and the problem of haemocompatibility still arises.

Lastly, it is an object of the invention to which Patent FR 2 672 217 relates, and which is illustrated in Figure 6, to auto-regulate a device 1 having a single needle 2. In the flow direction of the blood, the circuit 1 includes an arterial clamp 10, an arterial pump 14, an arterial expansion chamber 60, a treatment unit 3, a venous expansion chamber 50 and a venous pump 52. Each expansion chamber (50, 60) is connected to pressure regulating means (56 and 66) for maintaining an adjustable, substantially constant pressure in the chamber. The regulating means are pumps, which are controlled in order to take air in during an arterial phase, and which are controlled in order to the deliver air during a venous phase, the purpose of this being to maintain a constant pressure on either side of the filter 3 and to set a constant pressure inside the filter.

Another improvement has been proposed in Patent Application EP0392304, which relates to a single-needle dialysis machine including at least one blood container in the form of a flexible bag arranged between two support plates. One of the plates is fixed and the other can be moved, during the venous phase only, in order to control the emptying of the flexible bag. During the arterial phase, the bag becomes filled up to a certain high liquid limit and acts on the free mobile plate. During the venous phase, the bag is emptied by the pressure exerted by the controlled mobile plate on the entirely flexible bag, until a low liquid level is reached. The high and low pressure limits are detected by a pressure detector in the bag.

This device uses a flexible bag for holding blood and air, but it nevertheless has the problem of haemocompatibility.

Another proposal is found in Patent Application EP0462422, which discloses a device with at least one blood accumulator which, at least in the venous phase, is supplied with a force produced by an external instrument, so that a pressure essentially independent of its filled volume is formed on the inside. The accumulator is therefore inflated like a balloon during the arterial phase, and deflated during the venous phase. In this embodiment, the management of flow rate and pressure with a deformable flexible bag remains very difficult and not very accurate, especially as the pressure also turns out to be dependent on the elasticity of the bag.

Recurrent problems have thus been encountered for many years when producing the single-needle extracorporeal blood treatment devices of the prior art. These circuits are complex and expensive owing to the number of expansion chambers, pumps and bubble traps which are used, etc., and they are not very efficient in terms of the maximum blood flow which circulates and is therefore treated. Problems of haemocompatibility and coagulation, in particular owing to the air/blood interface in each reservoir, are still observed.

The problem of constant haemodynamic conditions also arises: the flow rate and pressure of blood, in particular, ought to be as constant as possible. It is furthermore desirable to regulate or auto-regulate the operation of the machine, without systematic intervention by an operator during the treatment session. These circuits are furthermore reserved for use in a single-needle mode, and cannot be used in a double-needle mode, particularly in the field of renal intensive care.

It is an object of the present application to provide a single-needle extracorporeal blood treatment device which is designed in a simple way, can be used in a device for the double-needle mode, is reliable, can be regulated and entails less coagulation. It is also an object to improve the haemodynamic conditions in order to obtain substantially constant blood flow rates and pressures.

**Description of the Invention:**

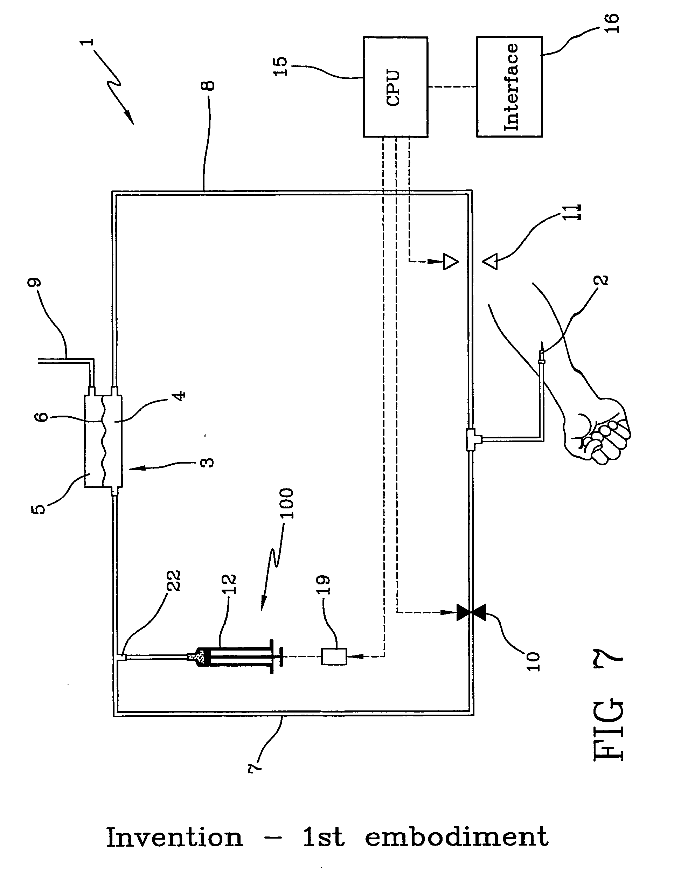

In order to achieve these objects, the invention provides an extracorporeal blood treatment device 1 (Fig. 7) intended to operate in a mode with a single needle 2, comprising:

- a treatment unit 3 having a first compartment 4 and a second compartment 5, which are separated by a semipermeable membrane 6,
- an extracorporeal blood circuit comprising a single needle 2, an intake line 7 in fluid communication with the first compartment 4 of the treatment unit 3, the first compartment 4, and a return line 8 in fluid communication with the first compartment 4 of the treatment unit 3,
- a purge line 9 at the outlet of the second compartment 5,
- a closure means (10, 11) acting on at least the intake line 7 and the return line 8 in order to generate the alternate sequence of blood intake and return,
- at least a first chamber 12 which is in fluid communication with the extracorporeal blood circuit and defines a first additional blood volume,
- the first chamber 12 having a variable total content,
- the first chamber being rigid and including at least one wall capable of being moved,
- the device including a means 19 acting on the chamber 12 in order to modify the volume of the chamber 12, making it possible to store blood during the arterial phase and release blood during the venous phase, and the first chamber including an opening to the outside, comprising a hydrophobic diaphragm (41) which is associated with the said opening.

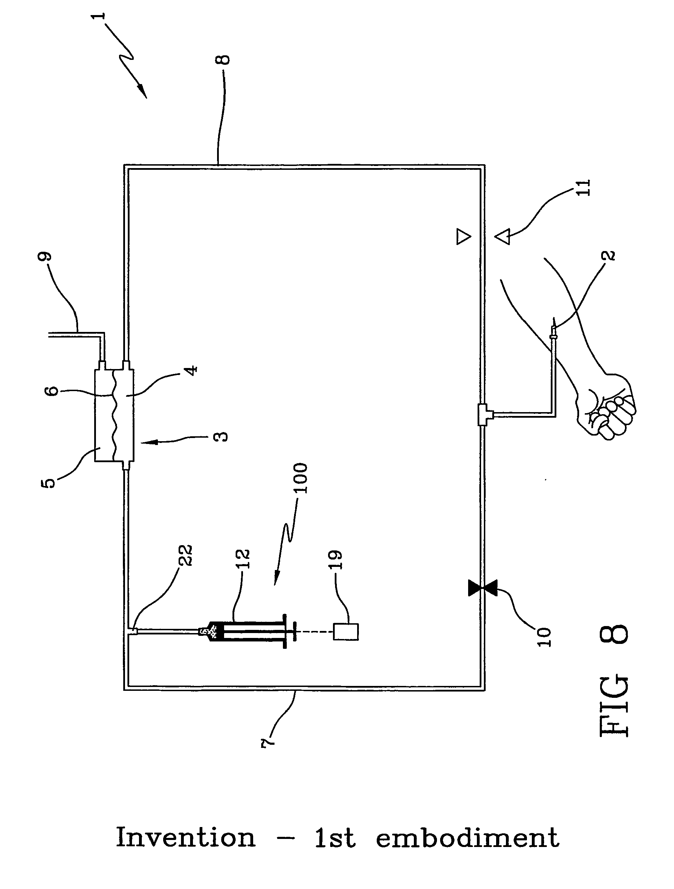

The invention also relates to a disposable device 100 (Fig. 8) for use in an extracorporeal blood circuit 1 with a single needle 2, comprising:

- a treatment unit 3 having a first compartment 4 and a second compartment 5, which are separated by a semipermeable membrane 6,
- an extracorporeal blood circuit comprising a single needle 2, an intake line 7, the first compartment 4 of the treatment unit 3 and a return line 8,
- at least a first chamber 12 for defining a first additional blood volume which includes a connection 22 in fluid communication with the intake line 7 or with the return line 8,
- the first chamber 12 having a variable total content,
- the first chamber 12 being rigid and including at least one wall capable of being moved, and the first chamber including an opening to the outside, comprising a hydrophobic diaphragm (41) which is associated with the said opening.

Other advantages and characteristics of the invention will become apparent on reading the description which follows.

## Brief Description of the Drawings:

Reference will be made to the appended drawings, in which:

Figures 1 to 4 represent prior-art circuits relating to the treatment of blood with a single-needle device;
Figure 5 represents the prior art described in Patent FR 2 548 906;
Figure 6 represents the prior art described in Patent FR 2 672 217;

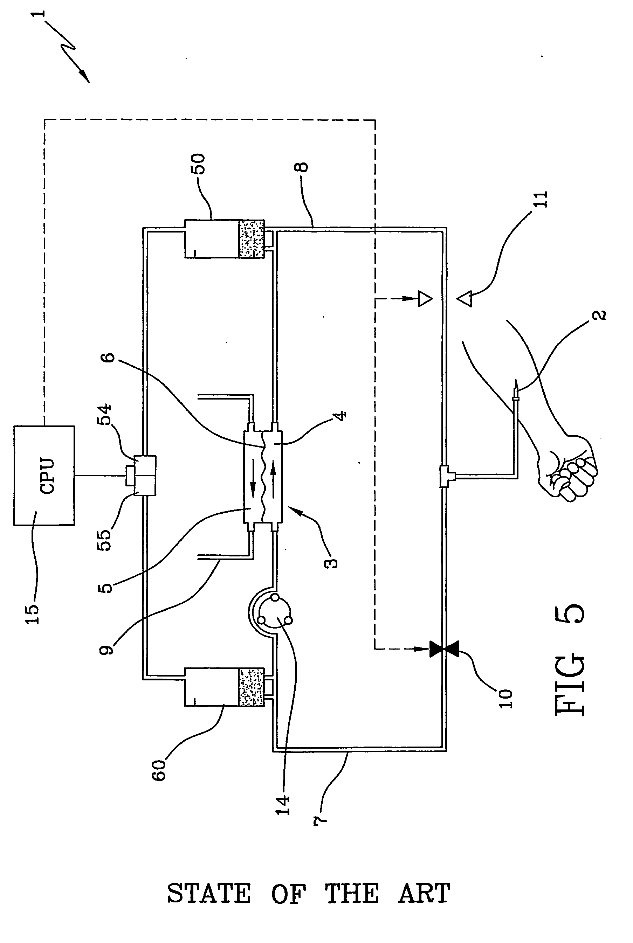

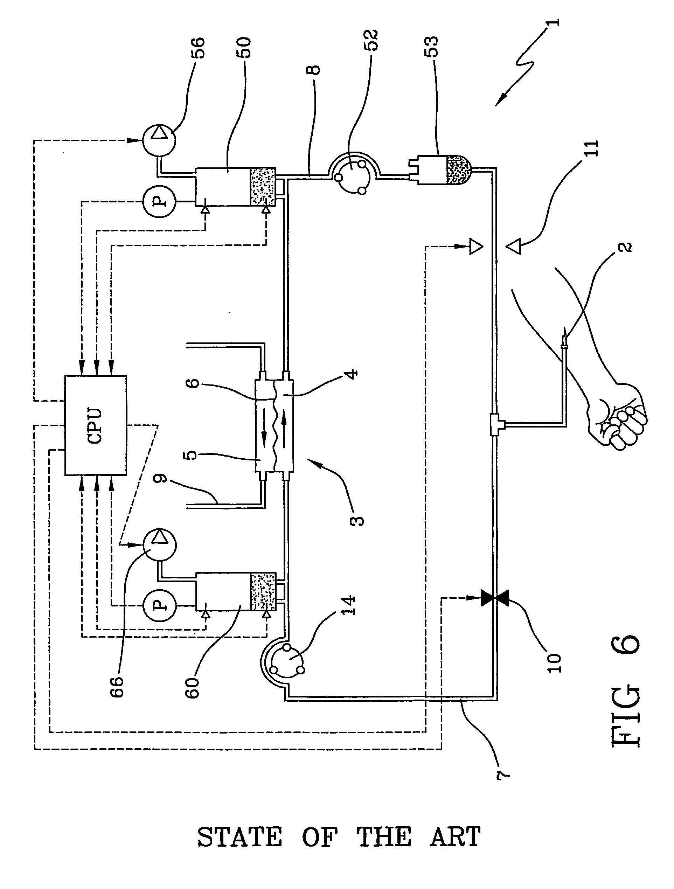

Figures 1 to 6 were discussed in the introduction to the prior art.
Figure 7 (respectively Figure 8) represents in a schematized way a first embodiment (respectively the corresponding disposable device) of the invention with one chamber;

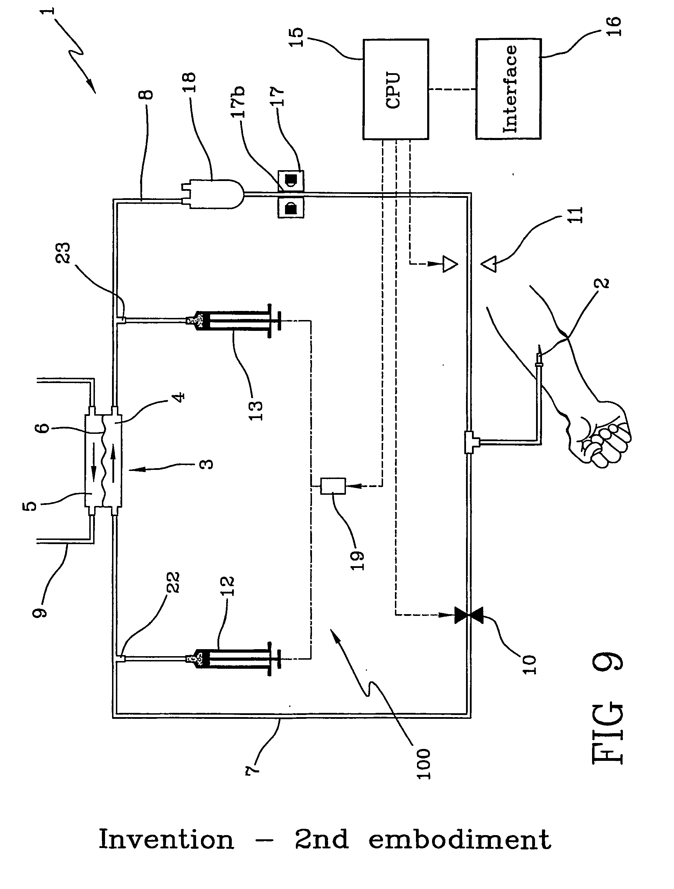

Figure 9 represents a second embodiment with two chambers in a schematized way;

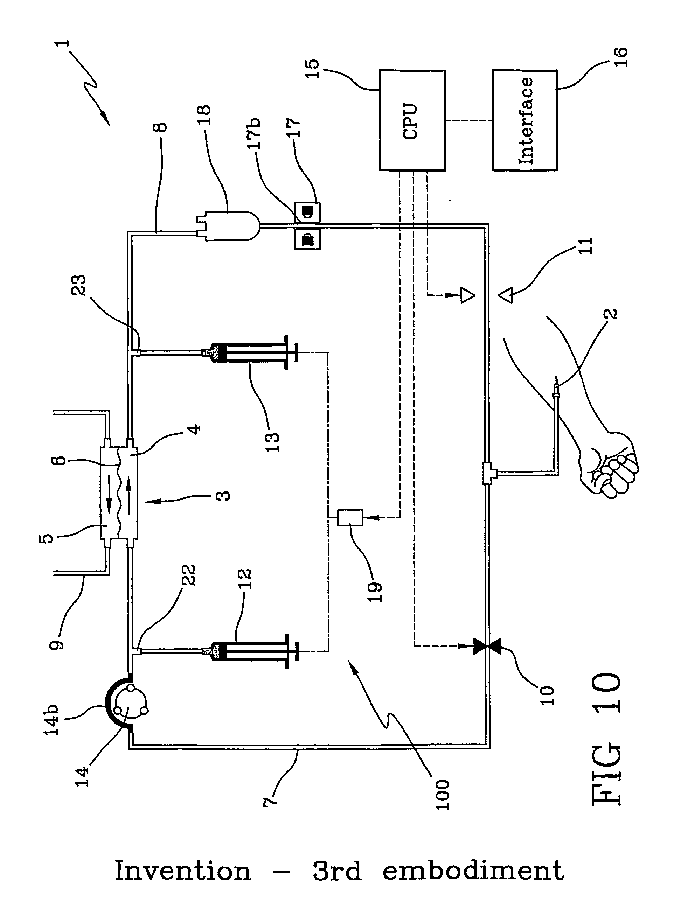

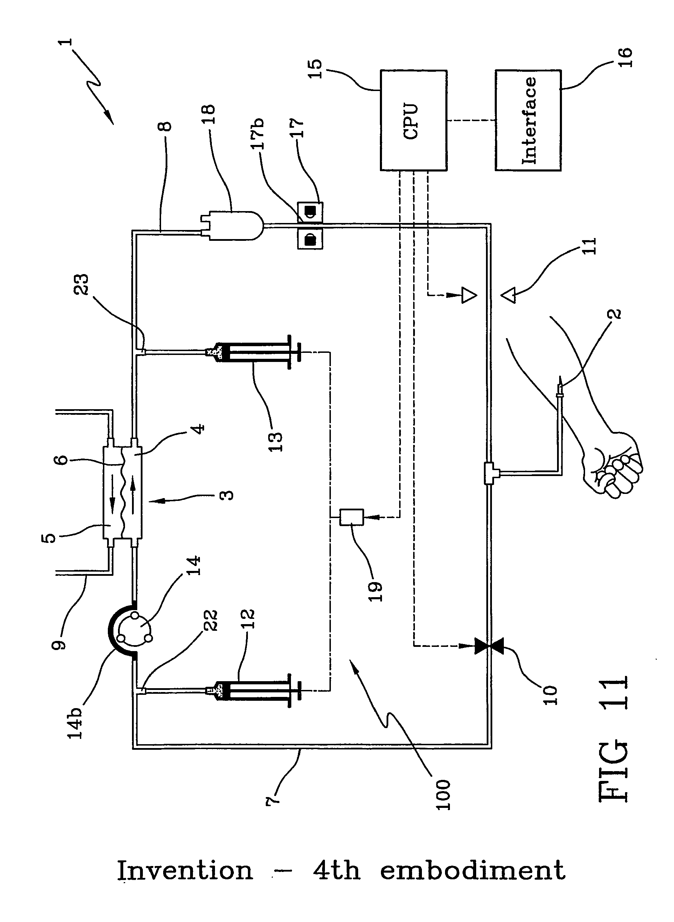

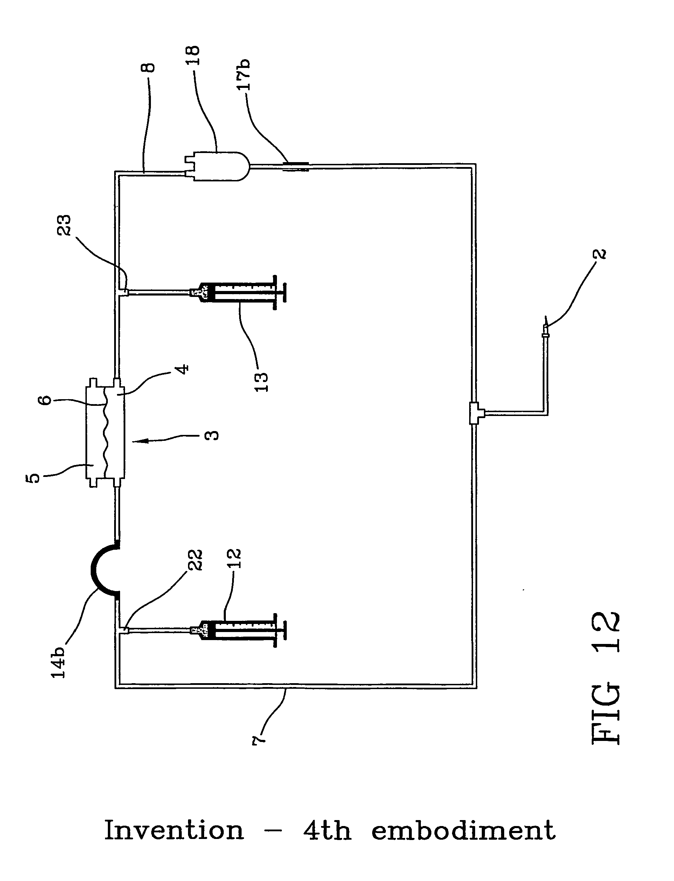

Figures 10 and 11 represent in a schematized way a third and a fourth embodiment with two chambers and a flow-rate regulating means (arbitrarily designed pump) positioned differently; Figure 12 represents the disposable device corresponding to the fourth embodiment;

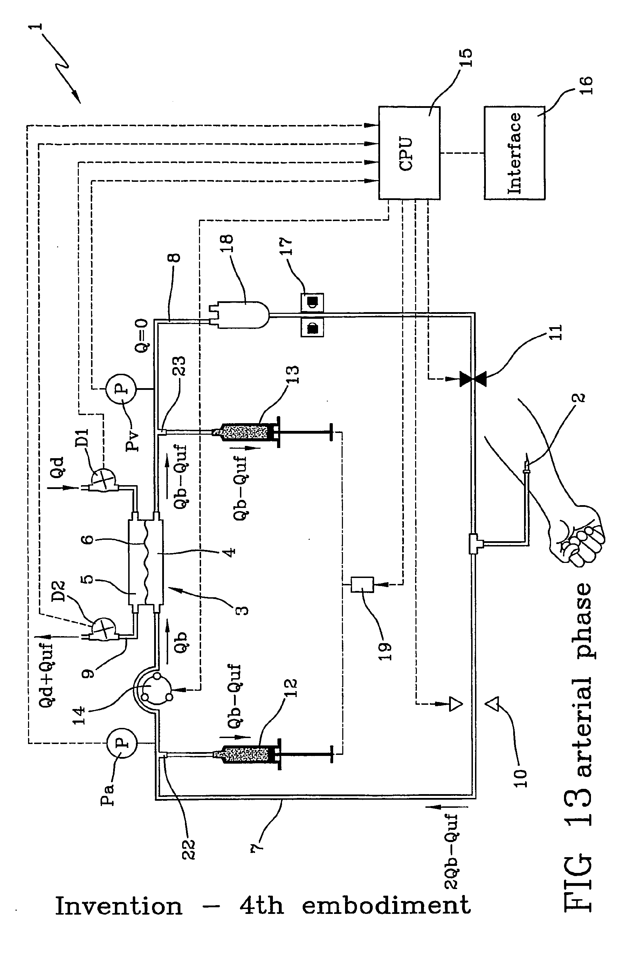

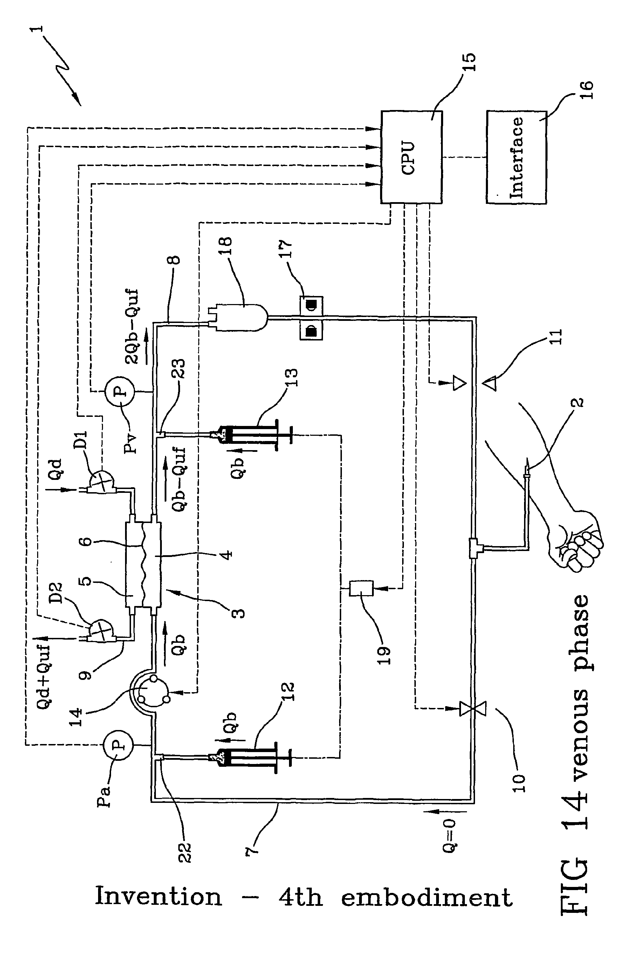

Figures 13 and 14 represent the operation of the device according to the fourth embodiment of the invention, respectively in an arterial phase and in a venous phase;

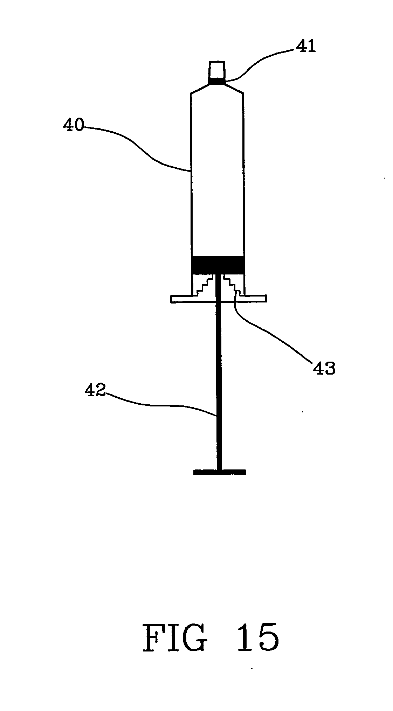

Figure 15 represents a syringe which may be used in each embodiment.

## Detailed Description of Embodiments of the Invention:

Figure 7 represents an extracorporeal blood treatment device 1 intended to operate in a mode with a single needle 2, comprising a treatment unit 3 having a first compartment 4 and a second compartment 5, which are separated by a semipermeable membrane 6, an extracorporeal blood circuit comprising a single needle 2, an intake line 7 in fluid communication with the first compartment 4 of the treatment unit 3, the first compartment 4, and a return line 8 in fluid communication with the first compartment 4 of the treatment unit 3, the first compartment 4, and a return line 8 in fluid communication with the first compartment 4 of the treatment unit 3, a purge line 9 at the outlet of the second compartment 5, a closure means (10, 11) acting on at least the intake line 7 and the return line 8 in order to generate the alternate sequence of blood intake and return, at least a first chamber 12 which is in fluid communication with the extracorporeal blood circuit and defines a first additional blood volume; the first chamber 12 having a variable total content, being rigid and including at least one wall capable of being moved; the device including a means 19 acting on the chamber 12 in order to modify the volume of the chamber 12. The chamber 12 will thus store blood during the arterial phase and release blood during the venous phase.

The chamber is rigid in so far as it can withstand the deformation forces liable to be induced by a pressure, in particular. The material could be plastic, glass, etc.

In this first embodiment, the first chamber 12 is in fluid communication with the intake line 7. As an alternative, the first chamber 12 is in fluid communication with the return line 8.

Furthermore, a second embodiment (Fig. 9) relates to the same device with a second chamber 13 which is in fluid communication with the extracorporeal blood circuit and defines a second additional blood volume, the said second chamber 13 having a variable total content making it possible to store blood during the arterial phase and release blood during the venous phase, this chamber 13 being rigid and including at least one wall capable of being moved. More particularly, the second chamber 13 may be in fluid communication with the return line 8 when the first chamber is in fluid communication with the intake line 7.

A fluid flow-rate regulating means 14 may be present and act on the extracorporeal blood circuit. This fluid flow-rate regulating means may include a pump, particularly a peristaltic pump. This pump 14 may act upstream of the first compartment 4 of the treatment unit. More particularly, this pump 14 acts upstream of the first chamber 12 (third embodiment, illustrated in Figure 10) or downstream of the first chamber 12 (fourth embodiment, illustrated in Figure 11). As an alternative, this pump 14 may act downstream of the first compartment 4 of the treatment unit. In this case, this pump 14 or its equivalent would act either upstream or downstream of the second chamber 13.

The closure means (10, 11) may be a pump, a valve, an active clamp or a combination of these instruments. Two clamps have been represented arbitrarily in the 4 embodiments which are illustrated, the first clamp 11 referred to as the "arterial clamp" acting on the intake line or arterial line 7, and the second clamp 12 referred to as the "venous clamp" acting on the return line or venous line 8.

An air detector 17 may be placed upstream of the part of the venous closure means (10, 11) acting on the return line 8. A part 17b of the return line will be operational with the air detector.

An air separator 18 may be inserted into the device. It may be connected downstream of the chamber or chambers (12, 13) and the treatment unit 3. The air separator is advantageously connected upstream of the part of the closure means (10, 11) acting on the return line 8, and immediately upstream of the optional air detector 17. The air separator 18 may also receive the air detector 17 on its own structure.
The air separator (18) may include a bubble trap. An air separator may also be inserted into each chamber (12, 13).

At least one of the chambers may be a rigid chamber with at least one wall intended to slide in the chamber. More particularly, one of the chambers may be a syringe. Such a syringe is advantageously positioned vertically with the needle upwards. These characteristics are illustrated in the first four embodiments.

The syringe may furthermore include an extensible diaphragm impermeable to bacteria, or to blood, of which at least one part is attached to the plunger and another part is attached to a circumference of the barrel of the syringe, so that it forms a barrier between the blood contained in the syringe and the ambient air. This makes it possible, in particular, to prevent contamination of the blood by air if a drop of blood were to pass between the sliding mobile wall and the barrel of the syringe. The diaphragm could be formed by gauze, cotton, etc.
The elastic diaphragm 43 impermeable to bacteria connects a point of the plunger to a circumference of the barrel towards the outer part of the latter. The diaphragm may be in the form of a concertina, for example, which can expand when the syringe is empty and contract when the syringe is full.
At least one of the chambers (12, 13) includes an opening to the outside, comprising a hydrophobic diaphragm (41) which is associated with the said opening and makes it possible to remove any undesirable air bubble present in the chamber (12, 13). This diaphragm may be placed as close as possible to the outlet of the syringe. It is represented at the top of the barrel. 40 forming the syringe in Figure 15, although it may be located anywhere on the chamber, on the mobile wall (plunger 42 in Fig. 15) or on the fixed wall.

In all the modes which are described, provision may be made for at least one chamber part (12, 13) to be transparent and include a scale for visual reading of the content. This allows the operator to immediately check the filling state of the chamber or chambers and to ascertain the "stroke volume" SV.

At least one chamber (12, 13) may be also be integrated inside the treatment unit 3. The two chambers (12, 13) advantageously have the same structure and the same maximum content. Once the equipment has been regulated, this makes it possible to distribute the stroke volume into two identical volumes throughout operation.

A calculation and control unit (CPU) 15 is provided for simultaneously controlling the closure means (10, 11) in order to alternate the arterial phase and the venous phase, and controlling the filling of at least one of the two chambers (12, 13) during the arterial phase and the discharge of at least one of the two chambers (12, 13) during the venous phase.

The means 19 acting on the chambers (12, 13) in order to modify the volume of the chambers (12, 13) includes at least one actuation means for varying the volume of the chamber by moving at least one chamber wall. It will, for example, include a plunger.

The means 19 comprises a motor coupled with the actuation means. In the embodiment which includes two identical syringes, the motor will be coupled to the plunger of each syringe. The coupling will be such that the two plungers are actuated simultaneously and in the same position.

The CPU 15 comprises elements for controlling the means. 19 acting on the chambers (12, 13) for modifying the volume of the chambers (12, 13) in order to fill at least one of the two chambers (12, 13) during the arterial phase, and to discharge at least one of the two chambers (12, 13) during the venous phase.

The CPU 15 comprises means for controlling the first fluid flow-rate regulating means 14 in order to ensure a substantially constant flow rate immediately downstream of the latter. This improves the quality of the blood treatment.

The CPU advantageously comprises means for controlling the means acting on the chambers (12, 13) for modifying the volume of the two chambers (12, 13) in order to set a volume in the first chamber 12 substantially equal to the volume in the second chamber 13 during operation of the device.

A user interface 16 is also provided, with means for receiving intended parameters, the CPU 15 comprising means for calculating control parameters from one or more planned machine parameters.

The device may also include:

- a user interface 16 having means for receiving intended parameters;
- measurement means for ascertaining measured parameters;
- in which the calculation and control means 15 comprises means for calculating control parameters from one or more intended parameters and one or more measured parameters, and for controlling the device as a function of them. The measurement means advantageously include pressure measurement means ($P_A$, $P_V$) for measuring the arterial pressure and the venous pressure. The arterial pressure may be measured upstream of the pump 14, and the venous pressure is the pressure at the outlet of the first compartment of the treatment unit (configuration in Figure 13). The measurement means may also include at least one flow meter (D1, D2) for ascertaining the ultrafiltration rate. A flow meter D1 may be provided on the fresh dialysate line and a flow meter D2 may be provided on the spent dialysate line, in order to ascertain the ultrafiltration rate, that is to say the flow rate of liquid extracted from the patient. At least the intended stroke volume $SV_{int}$ figures among the intended parameters.

**Operation of the Fourth Embodiment:**

The operation of an embodiment will be described in more detail with reference to Figures 13 and 14.

Before the start of the treatment, the two syringes 12 and 13 are empty and are coupled with the actuation means 19 "driver". If the extracorporeal blood treatment device 1 is initially in a double-needle mode, the operator will be able to connect the disposable according to the invention onto the machine, unlock the syringes and tap the single needle 2 into the patient's blood access.

As soon as the treatment has started, the peristaltic pump 14 is primed, a first arterial phase is initiated by making the arterial clamp 10 open and the venous clamp 11 close, the blood passes through the needle and the intake line 7, fills the first syringe 12 and enters the first compartment 4 of the treatment unit 3, then the treated blood leaves the compartment 4, passes through the return line and fills the second syringe 13. Throughout the arterial phase, the blood is both stored and purified (in part) through the treatment unit.

A budget of the flow rates can be determined easily, and this is reported on Figure 13. $Q_B$ denotes the blood flow rate in the first compartment 4 of the treatment unit 3, $Q_D$ denotes the dialysate flow rate in the second compartment 5 and $Q_{UF}$ denotes the ultrafiltration rate of the patient's fluid taking place through the membrane 6, passing from the first compartment 5 to the second compartment 4. The blood flow rate downstream of the treatment unit is accordingly reduced by $Q_{UF}$.

In the embodiment being described, the chambers are syringes with the same structure and the same maximum content, equal to the stroke volume (SV) divided by 2. It will be recalled that the stroke volume SV is the maximum volume taken from the patient during one cycle (during the arterial phase and the venous phase). The arterial stroke volume $SV_A$ (respectively the venous stroke volume $SV_v$) denotes the maximum volume taken from the patient and pumped through the "arterial" first chamber 12 (respectively the "venous" second chamber 13). The following equations are therefore satisfied for this embodiment: $SV_A = SV_v = SV/2$.

Once the syringes have reached a predetermined filling threshold, the venous phase is then initiate d by reverse control of the two clamps and return of the plungers of the syringes. The blood is accordingly released and returned to the patient.

**In a first possible control configuration of the fourth embodiment:**

Besides the stroke volume SV, the int ended parameters may include the intended blood flow rate $Q_{Bint}$ at the inlet of the first compartment 4.

The control parameters accordingly include at least the duration of the arterial phase $T_A$ and the duration of the venous phase $T_V$.

The budget of the flow rates in an arterial phase is illustrated in Figure 13, and makes it possible to derive the control parameter:

$$T_A = \frac{Vsyringe}{(Qsyringe)} = \frac{SV}{2 * (QBint - QUF)}$$

The following is likewise derived in a venous phase (see Figure 14):

$$T_V = \frac{Vsyringe}{(Qsyringe)} = \frac{SV}{2 * QBint}$$

As an alternative to choosing TA and TV, at least the actuation speed of the actuation means $V_{ACT}$ during the arterial phase, on the one hand, and during the venous phase, on the other hand, may be selected as control parameters. This is because the actuation speed can be derived from the flow rate and the corresponding phase duration. Measurement of the actuation time and the actuation speed of the syringes, or alternatively measurement of the volumes, or else the first of the two which pertains, may also be used as a reference for changing from one phase to another.

It should be recalled, however, that the chambers (12, 13) are airless chambers and behave somewhat like pumps.

The pressure on the circuit will therefore need to be regulated. To that end, the calculation and control unit includes means for regulating the blood flow rate $Q_B$ around the intended blood flow rate $Q_{Bint}$ as a function of the measured parameters $P_A$ and $P_V$. substantial variation around $Q_{Bint}$: If $P_V$ decreases, then $Q_B$ will be substantially increased, and vice versa.

In a venous phase, there may likewise be a pressure "conflict" between the "arterial" chamber 12 and the pump 14 (due to flow rates which do not match, taking $Q_{UF}$ into account). It will accordingly be necessary to measure the venous pressure $P_A$ and regulate the blood flow rate QB as a function of this measurement $P_A$ by substantial variation around $QB_{int}$: If $P_A$ decreases, then $Q_B$ will be substantially decreased, and vice versa.

This regulation makes it possible to refine the pressures $P_A$ and $P_V$ very effectively in order to maintain the haemodynamic conditions of constant pressure and flow rate.

**In a second possible control configuration of the fourth embodiment:**

Besides the stroke volume $SV_{int}$, the intended parameters may include the actuation speed $V_{ACTint}$ of the actuation means or, as an alternative to $V_{ACTint}$, the intended duration of the arterial phase $T_{Aint}$ and the intended duration of the venous phase $T_{Vint}$.

The control parameters accordingly include at least the blood flow rate $Q_B$.

The budget of the flow rates in an arterial phase is illustrated in Figure 13, and makes it possible to derive the control parameter:

$$Q_{Bart} = QUF + \frac{SV}{2 * TAint}$$

The following control parameter is likewise derived in a venous phase (see Figure 14):

$$Q_{Bvei} = \frac{SV}{2 * TVint}$$

In the same way as in the first control configuration, the pressure in the circuit will need to be regulated. To that end, the calculation and control unit includes means for regulating the actuation speed $V_{ACT}$ around the intended actuation speed $V_{ACTint}$ as a function of the measured parameters $P_A$ and $P_V$. As an alternative, the calculation and control unit will include means for regulating the duration of the arterial phase $T_A$ around the intended duration of the arterial phase $T_{Aint}$ and for regulating the duration of the venous phase $T_V$ around the intended duration of the venous phase $T_{Vint}$, as a function of the measured parameters $P_A$ and $P_V$.

As an alternative, it will be possible to work either with the flow rate of the pump 14 or with the flow rate of the syringes (12, 13), or with both, in order to regulate the arterial pressure $P_A$ and the venous pressure $P_V$.

The calculation and control unit 15 is also intended to comprise means for controlling the following modes of operation: haemodialysis, haemofiltration, haemodiafiltration.

The invention also relates to the disposable device (or "disposable") 100 corresponding to the embodiments of the various instruments described above. The disposable device of the first embodiment, illustrated in Figure 8, includes a treatment unit 3 having a first compartment 4 and a second compartment 5, which are separated by a semipermeable membrane 6; an extracorporeal blood circuit comprising a single needle 2, an intake line 7, the first compartment 4 of the treatment unit and a return line 8; at least a first chamber 12 for defining a first additional blood volume which includes a connection 22 in fluid communication with the intake line 7 or with the return line 8; the first chamber 12 having a variable total content and being rigid and including at least one wall capable of being moved. The first chamber 12 is advantageously in fluid communication with the intake line 7 via the said connection 22, which is fixed or removable. The disposable device of the fourth embodiment also includes a second chamber 13 for defining a second additional blood volume which includes a fixed or removable second connection 23 for placing the second chamber 13 in fluid communication with the return line 8, the second chamber 13 having a variable total content and being rigid and including at least one wall capable of being moved. The disposable device may include at least one part 14b capable of working with a flow-rate regulating means 14. This part 14b is placed on the intake line 7, downstream of the first connection 22. The disposable device may include a part 17b placed on the return line 8 and capable of working with an air detector 17. An air separator 18 may be connected onto the return line 8.

At least one of the chambers (12, 13) may be a rigid chamber with at least one wall intended to slide in the chamber, and will more particularly be a syringe. (The syringe may include an extensible diaphragm, which is impermeable to blood and of which at least one part is attached to the plunger and another part is attached to a circumference of the barrel of the syringe).

The syringe may moreover include an opening to the outside, comprising a hydrophobic diaphragm 41 which is associated with the said opening and makes it possible to remove any undesirable air bubble present in the chamber (12, 13). In all the modes, at least one part of the chamber or chambers (12, 13) may be transparent and include a scale for visual reading of the storage capacity.

At least one chamber (12, 13) may also be integrated inside the treatment unit 3.

The two storage means (12, 13) advantageously have the same structure and the same maximum content.

They may be placed side by side in order to facilitate their control by the actuation means. In the case of two syringes, they will be identical and placed side by side, and will have a common linear actuation means for the plungers.

The syringes may themselves be removable and fittable by virtue of two connectors 22 and 23 on the disposable device, which makes it possible to select and fit the syringes before the treatment, as a function of the intended stroke volume.

These controllable syringes with a variable total content are particularly advantageous because the storage of blood during single-needle operation obviates the use of the expansion chambers in the prior art. On the one hand, this obviation makes it possible to reduce coagulation owing to the absence of any air/blood interface, and makes it possible to avoid the compulsory presence of an air separator required downstream of the first chamber and upstream of the treatment unit. On the other hand, this obviation makes it possible to avoid having to ascertain and control the parameters of air pressure and air volume in the chambers. Indeed, level detectors or the like are no longer necessary for ascertaining the quantity of blood in the chamber: the position of the plunger or of the mobile partition of the chamber is easy to derive (from the maximum content and the position of the actuation means) and will give its instantaneous content. The syringes can furthermore be readily emptied again at the end of the venous phase, so that no blood will remain in the syringe, while maintaining accurate knowledge of the internal volume.

Placed side by side, the syringes furthermore fulfil the function of a pump and will make it possible to reduce the number of pumps needed in order to ensure a maximally constant, known and easily controllable blood flow rate. The flow rate of blood through the first compartment of the treatment unit is thus kept continuous and substantially constant.

The invention provides many advantages. It makes it possible:

- To obtain improved biocompatibility owing to a limited risk of blood coagulation;
- To change easily from a single-needle mode of operation to a double-needle mode of operation, and vice versa;
- To see and ascertain the volume of blood by a visual assessment of the quantity of blood;

To reduce the extracorporeal volume of blood (stroke volume) to be treated,

- To carry out easier integration of the machine,
- To limit costs by the restricted use of pumps and storage means, and by the possibility of changing between the two modes, single-needle and double-needle;
- to facilitate the procedure for the operator owing to a single connection.

**Claims**

1. Device for extracorporeal treatment of blood (1) intended to operate in a mode with a single needle (2), comprising:

   - a treatment unit (3) having a first compartment (4) and a second compartment (5), which are separated by a semipermeable membrane (6),
   - an extracorporeal blood circuit comprising a single needle (2), an intake line (7) in fluid communication with the first compartment (4) of the treatment unit (3), the first compartment (4), and a return line (8) in fluid communication with the first compartment (4) of the treatment unit (3),
   - a purge line (9) at the outlet of the second compartment (5),
   - a closure means (10, 11) acting on at least the intake line (7) and the return line (8) in order to generate the alternate sequence of blood intake and return,
   - at least a first chamber (12) which is in fluid communication with the extracorporeal blood circuit and defines a first additional blood volume, the first chamber (12) having a variable total content;
   - the first chamber (12) is rigid and includes at least one wall capable of being moved,
   - the device includes a means (19) acting on the chamber (12) in order to modify the volume of the chamber (12), making it possible to store blood during the arterial phase and release blood during the venous phase,

   **characterized in that**:

   the first chamber (12) includes an opening to the outside, comprising a hydrophobic diaphragm (41) which is associated with the said opening and makes it possible to remove any undesirable air bubble present in the chamber (12).

2. Device according to Claim 1, **characterized in that** the first chamber (12) is in fluid communication with the intake line (7).

3. Device according to Claim 1, **characterized in that** the first chamber (12) is in fluid communication with the return line (8).

4. Device according to Claim 1, comprising a second chamber (13) which is in fluid communication with the extracorporeal blood circuit and defines a second additional blood volume, the said second chamber (13) being rigid and including at least one wall capable of being moved; the second chamber having a variable total content making it possible to store blood during the arterial phase and release blood during the venous phase.

5. Device according to Claim 2 and the preceding claim, in which the second chamber (13) is in fluid communication with the return line (8).

6. Device according to one of the preceding claims comprising a fluid flow-rate regulating means (14) acting on the extracorporeal blood circuit, in which the said fluid flow-rate regulating means includes a pump.

7. Device according to the preceding claim, in which the said fluid flow-rate regulating means (14) acts upstream of the first compartment (4) of the treatment unit.

8. Device according to the preceding claim, in which the said fluid flow-rate regulating means (14) acts upstream of the first chamber (12).

9. Device according to Claim 7, in which the said fluid flow-rate regulating means (14) acts downstream of the first chamber (12).

10. Device according to Claim 6, in which the said fluid flow-rate regulating means (14) acts downstream of the first compartment (4) of the treatment unit.

11. Device according to Claim 10, in which the said fluid flow-rate regulating means (14) acts upstream of the second chamber (13).

12. Device according to Claim 10, in which the said fluid flow-rate regulating means (14) acts downstream of the second chamber (13).

13. Device according to one of the preceding claims, including at least one air detector (17) placed upstream of the part of the venous closure means (10, 11) acting on the return line (8).

14. Device according to one of the preceding claims, including at least one air separator (18).

15. Device according to the preceding claim, in which the air separator is connected downstream of the chamber or chambers (12, 13) and the treatment unit (3) and immediately upstream of the air detector (17).

16. Device according to one of Claims 14 to 15, in which the air separator (18) includes a bubble trap.

17. Device according to one of Claims 14 and 15, in which the air separator (18) is inserted into each chamber (12, 13).

18. Device according to one of the preceding claims, in which at least one of the chambers (12, 13) is a rigid chamber with at least one wall intended to slide in the chamber.

19. Device according to the preceding claim, in which at least one of the chambers is a syringe.

20. Device according to the preceding claim, in which at least one syringe is positioned vertically with the outlet of the needle upwards.

21. Device according to Claim 19 or 20, in which the syringe includes an extensible diaphragm impermeable to bacteria, of which at least one part is attached to the plunger and another part is attached to a circumference of the barrel of the syringe.

22. Device according to one of claims 4 to the preceding, in which the second chamber (13) includes an opening to the outside, comprising a hydrophobic diaphragm (41) which is associated with the said opening and makes it possible to remove any undesirable air bubble present in the second chamber (13).

23. Device according to one of Claims 19 to 22, in which at least one chamber part (12, 13) is transparent and includes a scale for visual reading of the content.

24. Device according to one of the preceding claims, in which at least one chamber (12, 13) is integrated inside the treatment unit (3).

25. Device according to one of Claims 19 to 24, in which the two chambers (12, 13) have the same structure and the same maximum content.

26. Device according to one of the preceding claims, comprising a calculation and control unit (15) for.

    a. simultaneously controlling the closure means (10, 11) in order to alternate the arterial phase and the venous phase, and
    b. controlling the filling of at least one of the two chambers (12, 13) during the arterial phase and the discharge of at least one of the two chambers (12, 13) during the venous phase.

**27.** Device according to one of the preceding claims, in which the means (19) acting on the chambers (12, 13) in order to modify the volume of the chambers (12, 13) includes an actuation means for varying the volume of the chamber by moving at least one chamber wall, in which case this means may include a plunger.

**28.** Device according to the preceding claim, in which the said means (19) comprises a motor coupled with the actuation means.

**29.** Device according to one of Claims 26 to 28, in which the calculation and control unit (15) comprises means for controlling the means (19) acting on the chambers (12, 13) to modify the volume of the chambers (12, 13) in order to fill at least one of the two chambers (12, 13) during the arterial phase, and to discharge at least one of the two chambers (12, 13) during the venous phase.

**30.** Device according to one of Claims 26 to 29, in which the calculation and control unit (15) comprises means for controlling the first fluid flow-rate regulating means (14) in order to ensure a substantially constant flow rate immediately downstream of the latter.

**31.** Device according to one of Claims 26 to 30, in which the calculation and control unit (15) comprises means for controlling the means for modifying the volume of the two chambers (12, 13) in order to set a volume in the first chamber (12) substantially equal to the volume in the second chamber (13) during operation of the device.

**32.** Device according to one of Claims 26 to 31, comprising:

   i. a user interface (16) having means for receiving intended parameters;
   ii. measurement means for ascertaining measured parameters;
   iii. in which the calculation and control means (15) comprises means for calculating control parameters from one or more intended parameters and one or more measured parameters, and for controlling the device as a function of them.

**33.** Device according to the preceding claim, in which the measurement means include pressure measurement means $(P_A, P_V)$ for measuring the arterial pressure and the venous pressure.

**34.** Device according to the preceding claim, in which the measurement means include at least one flow meter (D1, D2) for ascertaining the ultrafiltration rate.

**35.** Device according to Claim 33 or 34, in which the intended parameters include at least the intended stroke volume $SV_{int}$.

**36.** Device according to one of Claims 32 to 35, in which the intended parameters include the intended blood flow rate $Q_{Bint}$ at the inlet of the first compartment (4).

**37.** Device according to the preceding claim, in which the control parameters include at least the duration of the arterial phase $T_A$ and the duration of the venous phase $T_V$.

**38.** Device according to Claim 36, in which the control parameters include at least the actuation speed of the actuation means $V_{ACT}$.

**39.** Device according to Claim 37 or 38, in which the calculation and control unit includes means for regulating the blood flow rate $Q_B$ around the intended blood flow rate $Q_{Bint}$ as a function of the measured parameters $P_A$ and $P_V$.

**40.** Device according to one of Claims 32 to 35, in which the intended parameters include the intended actuation speed $V_{ACTint}$ of the actuation means.

**41.** Device according to one of Claims 32 to 35, in which the intended parameters include the intended duration of the arterial phase $T_{Aint}$ and the intended duration of the venous phase $T_{Vint}$.

**42.** Device according to one of Claims 40 and 41, in which the control parameters include at least the blood flow rate $Q_B$.

**43.** Device according to Claim 42, in which the calculation and control unit includes means for regulating the actuation speed $V_{ACT}$ around the intended actuation speed $V_{ACTint}$ as a function of the measured parameters $P_A$ and $P_V$.

**44.** Device according to Claim 42, in which the calculation and control unit includes means for regulating the duration of the arterial phase $T_A$ around the intended arterial phase duration $T_{Aint}$ and for regulating the duration of the venous phase $T_V$ around the intended venous phase duration $T_{Vint}$ as a function of the measured parameters $P_A$ and $P_V$.

**45.** Device according to any one of Claims 26 to 34, in which the calculation and control unit includes means for controlling the following modes of operation: haemodialysis, haemofiltration, haemodiafiltration.

**46.** Disposable device (100) for use in an extracorporeal blood circuit (1) with a single needle (2), comprising:

a. a treatment unit (3) having a first compartment (4) and a second compartment (5), which are separated by a semipermeable membrane (6);
b. an extracorporeal blood circuit comprising a single needle (2), an intake line (7), the first compartment (4) of the treatment unit (3) and a return line (8);
c. at least a first chamber (12) for defining a first additional blood volume which includes a connection (22) in fluid communication with the intake line (7) or with the return line (8),
d. the first chamber (12) having a variable total content,
e. the first chamber (12) being rigid and including at least one wall capable of being moved, **characterized in that**
f. the first chamber (12) includes an opening to the outside, comprising a hydrophobic diaphragm (41) which is associated with the said opening.

**47.** Disposable device according to the preceding claim, in which the first chamber (12) is in fluid communication with the intake line (7) via the said connection (22), which is fixed or removable.

**48.** Disposable device according to the preceding claim, comprising a second chamber (13) for defining a second additional blood volume which includes a fixed or removable second connection (23) for placing the second chamber (13) in fluid communication with the return line (8), the second chamber (13) having a variable total content, the second chamber (13) being rigid and including at least one wall capable of being moved.

**49.** Disposable device according to one of Claims 46 to 48, including at least one part (14b) capable of working with a flow-rate regulating means (14).

**50.** Disposable device according to the preceding claim, in which the said part (14b) is placed on the intake line (7), downstream of the first connection (22).

**51.** Disposable device according to one of Claims 46 to 50, including a part (17b) placed on the return line (8) and capable of working with an air detector (17).

**52.** Disposable device according to one of Claims 46 to 50, including at least one air separator (18) connected onto the return line (8).

**53.** Disposable device according to one of Claims 46 to 52, in which at least one of the chambers (12, 13) is a rigid chamber with at least one wall intended to slide in the chamber.

**54.** Disposable device according to the preceding claim, in which at least one chamber (12, 13) is a syringe.

**55.** Disposable device according to the preceding claim, in which the syringe includes an extensible diaphragm, which is impermeable to blood and of which at least one part is attached to the plunger and another part is attached to a circumference of the barrel of the syringe.

**56.** Disposable device according to one of Claims 48 to the preceding claim, in which the second chamber (13) includes an opening to the outside, comprising a hydrophobic diaphragm (41) which is associated with the said opening.

**57.** Disposable device according to one of Claims 46 to the preceding claim, in which at least one part of the chamber or chambers (12, 13) is transparent and includes a scale for visual reading of the storage capacity.

**58.** Disposable device according to one of Claims 46 to the preceding claim, in which at least one chamber (12, 13) is integrated inside the treatment unit (3).

**59.** Disposable device according to one of Claims 46 to the preceding claim, in which the two storage means (12, 13) have the same structure and the same maximum content.

## Patentansprüche

**1.** Vorrichtung zur extrakorporalen Blutbehandlung (1), die zum Betrieb mit einer Einzelnadel (2) vorgesehen ist, umfassend:

- eine Behandlungseinheit (3) mit einem ersten Abteil (4) und einem zweiten Abteil (5), die durch eine semipermeable Membran (6) voneinander getrennt sind,
- einen extrakorporalen Blutkreislauf umfassend eine Einzelnadel (2), eine Zuführungsleitung (7) in Fluidverbindung mit dem ersten Abteil (4) der Behandlungseinheit (3), den ersten Abteil (4), und eine Rückgabeleitung (8) in Fluidverbindung mit dem ersten Abteil (4) der Behandlungseinheit (3),
- eine Ablaufleitung (9) am Ausgang des zweiten Abteils (5),
- ein Verschlussmittel (10, 11), das auf mindestens die Zuführungsleitung (7) und die Rückgabeleitung (8) wirkt, um die alternierende Reihenfolge von Blutzuführung und - rückgabe zu erzeugen,
- mindestens eine erste Kammer (12), die in Fluidverbindung mit dem extrakorporalen Blutkreislauf steht und ein erstes Blutzusatzvolumen definiert, wobei die erste Kammer (12) einen veränderbaren Gesamtgehalt aufweist;
- wobei die erste Kammer (12) steif ist und mindestens eine bewegliche Wand umfasst;
- wobei die Vorrichtung ein Mittel (19) umfasst, das auf die Kammer (12) wirkt, um das Volumen der Kammer (12) zu ändern, so daß das Blut während des arteriellen Schrittes gespeichert und während des venösen Schrittes abgegeben werden kann,

**dadurch gekennzeichnet, daß**
die erste Kammer (12) eine Öffnung nach außen mit einer hydrophoben Scheidewand (41) umfasst, die mit der benannten Öffnung assoziiert ist und die Entfernung von eventuellen, ungewünschten Luftblasen in der Kammer (12) ermöglicht.

**2.** Vorrichtung nach Anspruch 1, **dadurch gekennzeichnet, daß** die erste Kammer (12) in Fluidverbindung mit der Zuführungsleitung (7) steht.

**3.** Vorrichtung nach Anspruch 1, **dadurch gekennzeichnet, daß** die erste Kammer (12) in Fluidverbindung mit der Rückgabeleitung (8) steht.

**4.** Vorrichtung nach Anspruch 1, umfassend eine zweite Kammer (13), die in Fluidverbindung mit dem extrakorporalen Blutkreislauf steht und ein zweites Blutzusatzvolumen definiert, wobei die benannte zweite Kammer (13) steif ist und mindestens eine bewegliche Wand umfasst; wobei die zweite Kammer einen veränderbaren Gesamtgehalt aufweist, so daß das Blut während des arteriellen Schrittes gespeichert und während des venösen Schrittes abgegeben werden kann.

**5.** Vorrichtung nach Anspruch 2 and dem vorhergehenden Anspruch, worin die zweite Kammer (13) in Fluidverbindung mit der Rückgabeleitung (8) steht.

**6.** Vorrichtung nach einem der vorhergehenden Ansprüche, umfassend ein Fluidflussmengeeinstellmittel (14), das auf den extrakorporalen Blutkreislauf wirkt, worin das benannte Fluidflussmengeeinstellmittel eine Pumpe umfasst.

**7.** Vorrichtung nach dem vorhergehenden Anspruch, worin das benannte Fluidflussmengeeinstellmittel (14) aufwärts von dem ersten Abteil (4) der Behandlungseinheit wirkt.

**8.** Vorrichtung nach dem vorhergehenden Anspruch, worin das benannte Fluidflussmengeeinstellmittel (14) aufwärts von der ersten Kammer (12) wirkt.

**9.** Vorrichtung nach Anspruch 7, worin das benannte Fluidflussmengeeinstellmittel (14) abwärts von der ersten Kammer (12) wirkt.

10. Vorrichtung nach Anspruch 6, worin das benannte Fluidflussmengeeinstellmittel (14) abwärts von dem ersten Abteil (4) der Behandlungseinheit wirkt.

11. Vorrichtung nach Anspruch 10, worin das benannte Fluidflussmengeeinstellmittel (14) aufwärts von dem zweiten Kammer (13) wirkt.

12. Vorrichtung nach Anspruch 10, worin das benannte Fluidflussmengeeinstellmittel (14) abwärts von dem zweiten Kammer (13) wirkt.

13. Vorrichtung nach einem der vorhergehenden Ansprüche, umfassend mindestens einen Luftdetektor (17), der sich aufwärts von dem Abschnitt des venösen Verschlussmittels (10, 11), der auf die Rückgabeleitung (8) wirkt, befindet.

14. Vorrichtung nach einem der vorhergehenden Ansprüche, umfassend mindestens einen Luftabscheider (18).

15. Vorrichtung nach dem vorhergehenden Anspruch, worin der Luftabscheider abwärts von der Kammer oder den Kammern (12, 13) und von der Behandlungseinheit (3) und unmittelbar aufwärts von dem Luftdetektor (17) angeschlossen ist.

16. Vorrichtung nach einem der Ansprüche 14 bis 15, worin der Luftabscheider (18) einen Blasenfänger umfasst.

17. Vorrichtung nach einem der Ansprüche 14 und 15, worin der Luftabscheider (18) in jeder Kammer (12, 13) installiert ist.

18. Vorrichtung nach einem der vorhergehenden Ansprüche, worin mindestens eine der Kammern (12, 13) eine steife Kammer mit mindestens einer Wand, die zum Gleiten in der Kammer vorgesehen ist, ist.

19. Vorrichtung nach dem vorhergehenden Anspruch, worin mindestens eine der Kammern eine Spritze ist.

20. Vorrichtung nach dem vorhergehenden Anspruch, worin mindestens eine Spritze vertikal positioniert ist, wobei der Nadelausgang nach oben ausgerichtet ist.

21. Vorrichtung nach Anspruch 19 oder 20, worin die Spritze eine verlängerbare, bakterienundurchlässige Scheidewand ist, deren mindestens eine Abschnitt am Kolben befestigt ist und ein anderer Abschnitt an einem Kreisumfang des Spritzenzylinders befestigt ist.

22. Vorrichtung nach einem der Ansprüche 4 bis zum vorhergehenden Anspruch, worin die zweite Kammer (13) eine Öffnung nach außen umfasst, umfassend eine hydrophobe Scheidewand (41), die mit der benannten Öffnung assoziiert ist und die Entfernung von eventuellen, ungewünschten Luftblasen in der zweite Kammer (13) ermöglicht.

23. Vorrichtung nach einem der Ansprüche 19 bis 22, worin mindestens einer Kammerabschnitt (12, 13) transparent ist und eine Skala zum visuellen Lesen des Gehaltes umfasst.

24. Vorrichtung nach einem der vorhergehenden Ansprüche, worin mindestens eine Kammer (12, 13) in die Behandlungseinheit (3) integriert ist.

25. Vorrichtung nach einem der Ansprüche 19 bis 24, worin die beide Kammern (12, 13) dieselbe Struktur und denselben maximalen Gehalt aufweisen.

26. Vorrichtung nach einem der vorhergehenden Ansprüche, umfassend eine Rechen- und Steuereinheit (15) zur:

    a. gleichzeitigen Steuerung des Verschlussmittels (10, 11), um den arteriellen und den venösen Schritt zu alternieren, und
    b. Steuerung der Befüllung von mindestens einer der beiden Kammern (12, 13) während des arteriellen Schrittes und der Entleerung von mindestens einer der beiden Kammern (12, 13) während des venösen Schrittes.

27. Vorrichtung nach einem der vorhergehenden Ansprüche, worin das Mittel (19), das auf die Kammern (12, 13) zum Verändern des Volumens der Kammern (12, 13) wirkt, ein Betätigungsmittel zum Verändern des Volumens der Kammer durch Bewegung von mindestens einer Kammerwand umfasst, wobei dieses Mittel einen Kolben umfassen kann.

28. Vorrichtung nach dem vorhergehenden Anspruch, worin das benannte Mittel (19) umfasst einen mit dem Betätigungsmittel verbundenen Motor.

29. Vorrichtung nach einem der Ansprüche 26 bis 28, worin die Rechen- und Steuereinheit (15) Mittel zur Steuerung des Mittels (19), das auf die Kammern (12, 13) zum Verändern des Volumens der Kammern (12, 13) wirkt, umfasst, um mindestens eine der beiden Kammern (12, 13) während des arteriellen Schrittes zu befüllen und mindestens eine der beiden Kammern (12, 13) während des venösen Schrittes zu entleeren.

30. Vorrichtung nach einem der Ansprüche 26 bis 29, worin die Rechen- und Steuereinheit (15) Mittel zur Steuerung des ersten Fluidflussmengeeinstellmittels (14) umfasst, um eine wesentlich konstante Flussmenge unmittelbar abwärts von dem letzteren zu gewährleisten.

31. Vorrichtung nach einem der Ansprüche 26 bis 30, worin die Rechen- und Steuereinheit (15) Mittel zur Steuerung des Mittels zum Verändern des Volumens der beiden Kammern (12, 13) umfasst, um ein Volumen in der ersten Kammer (12) wesentlich so hoch wie das Volumen in der zweiten Kammer (13) während des Betriebs der Vorrichtung einzustellen.

32. Vorrichtung nach einem der Ansprüche 26 bis 31, umfassend:

   i. eine Benutzerschnittstelle (16) mit Mitteln zum Empfang von vorgesehenen Parametern;
   ii. Messmittel zur Bestimmung von gemessenen Parametern;
   iii. worin das Rechen- und Steuermittel (15) Mittel zur Berechnung von Steuerparametern ausgehend von einem oder mehreren der vorgesehenen Parameter und von einem oder mehreren der gemessenen Parameter, und zur Steuerung der Vorrichtung abhängig davon umfasst.

33. Vorrichtung nach dem vorhergehenden Anspruch, worin die Messmittel Druckmessmittel ($P_A$, $P_V$) zur Messung des arteriellen Drucks und des venösen Drucks umfassen.

34. Vorrichtung nach dem vorhergehenden Anspruch, worin die Messmittel mindestens einen Durchflussmesser (D1, D2) zur Bestimmung der Ultrafiltrationsflussmenge umfassen.

35. Vorrichtung nach Anspruch 33 oder 34, worin die vorgesehenen Parameter mindestens das vorgesehene Herzschlagvolumen $SV_{int}$ umfassen.

36. Vorrichtung nach einem der Ansprüche 32 bis 35, worin die vorgesehenen Parameter die vorgesehene Blutflussmenge $Q_{Bint}$ am Eingang des ersten Abteils (4) umfassen.

37. Vorrichtung nach dem vorhergehenden Anspruch, worin die Steuerparameter mindestens die Dauer des arteriellen Schrittes $T_A$ und die Dauer des venösen Schrittes $T_V$ umfassen.

38. Vorrichtung nach Anspruch 36, worin die Steuerparameter mindestens die Betätigungsgeschwindigkeit des Betätigungsmittels $V_{ACT}$ umfassen.

39. Vorrichtung nach Anspruch 37 oder 38, worin die Rechen- und Steuereinheit Mittel zur Einstellung der Blutflussmenge $Q_B$ um die vorgesehene Blutflussmenge $Q_{Bint}$ abhängig von den gemessenen Parametern $P_A$ und $P_V$ umfasst.

40. Vorrichtung nach einem der Ansprüche 32 bis 35, worin die vorgesehenen Parameter die vorgesehene Betätigungsgeschwindigkeit $V_{ACTint}$ des Betätigungsmittels umfassen.

41. Vorrichtung nach einem der Ansprüche 32 bis 35, worin die vorgesehenen Parameter die vorgesehene Dauer des arteriellen Schrittes $T_{Aint}$ und die vorgesehene Dauer des venösen Schrittes $T_{Vint}$ umfassen.

42. Vorrichtung nach einem der Ansprüche 40 und 41, worin die Steuerparameter mindestens die Blutflussmenge $Q_B$ umfassen.

43. Vorrichtung nach Anspruch 42, worin die Rechen- und Steuereinheit Mittel zur Einstellung der Betätigungsgeschwindigkeit $V_{ACT}$ um die vorgesehene Betätigungsgeschwindigkeit $V_{ACTint}$ abhängig von den gemessenen Parametern $P_A$ und $P_V$ umfasst.

44. Vorrichtung nach Anspruch 42, worin die Rechen- und Steuereinheit Mittel zur Einstellung der Dauer des arteriellen Schrittes $T_A$ um die vorgesehene Dauer des arteriellen Schrittes $T_{Aint}$ und zur Einstellung der Dauer des venösen Schrittes $T_V$ um die vorgesehene Dauer des venösen Schrittes $T_{Vint}$ abhängig von den gemessenen Parametern $P_A$ und $P_V$ umfasst.

45. Vorrichtung nach einem der Ansprüche 26 bis 34, worin die Rechen- und Steuereinheit Mittel zur Steuerung der folgenden Betriebsarten: Hämodialyse, Hämofiltration, Hämodiafiltration, umfasst.

46. Einwegvorrichtung (100) zur Verwendung in einem extrakorporalen Blutkreislauf (1) mit einer Einzelnadel (2), umfassend:

    a. eine Behandlungseinheit (3) mit einem ersten Abteil (4) und einem zweiten Abteil (5), die durch eine semipermeable Membran (6) voneinander getrennt sind;
    b. einen extrakorporalen Blutkreislauf umfassend eine Einzelnadel (2), eine Zuführungsleitung (7), den ersten Abteil (4) der Behandlungseinheit (3), und eine Rückgabeleitung (8);
    c. mindestens eine erste Kammer (12) zur Definition eines ersten Blutzusatzvolumens, die einen Anschluss (22) in Fluidverbindung mit der Zuführungsleitung (7) oder mit dem Rückgabeleitung (7) umfasst,
    d. wobei die erste Kammer (12) einen veränderbaren Gesamtgehalt aufweist,
    e. wobei die erste Kammer (12) steif ist und mindestens eine bewegliche Wand umfasst, **dadurch gekennzeichnet, daß**
    f. die erste Kammer (12) eine Öffnung nach außen umfasst, umfassend eine hydrophobe Scheidewand (41), die mit dem benannten Öffnung assoziiert ist.

47. Einwegvorrichtung nach dem vorhergehenden Anspruch, worin die erste Kammer (12) in Fluidverbindung mit der Zuführungsleitung (7) durch die benannte Verbindung (22) steht, die fest oder entfernbar ist.

48. Einwegvorrichtung nach dem vorhergehenden Anspruch, umfassend eine zweite Kammer (13) zur Definition eines zweiten Blutzusatzvolumens, die einen festen oder entfernbaren zweiten Anschluss (23) zum Setzen der zweiten Kammer (13) in Fluidverbindung mit der Rückgabeleitung (8) umfasst, wobei die zweite Kammer (13) einen veränderbaren Gesamtgehalt aufweist, wobei die zweite Kammer (13) steif ist und mindestens eine bewegliche Wand umfasst.

49. Einwegvorrichtung nach einem der Ansprüche 46 bis 48, umfassend mindestens einen Abschnitt (14b), der mit einem Flussmengeeinstellmittel (14) zusammenarbeiten kann.

50. Einwegvorrichtung nach dem vorhergehenden Anspruch, worin der benannte Abschnitt (14b) sich an der Zuführungsleitung (7) abwärts von dem ersten Anschluss (22) befindet.

51. Einwegvorrichtung nach einem der Ansprüche 46 bis 50, umfassend einen Abschnitt (17b), die sich an der Rückgabeleitung (8) befindet und mit einem Luftdetektor (17) zusammenarbeiten kann.

52. Einwegvorrichtung nach einem der Ansprüche 46 bis 50, umfassend mindestens einen Luftabscheider (18), der an die Rückgabeleitung angeschlossen (8) ist.

53. Einwegvorrichtung nach einem der Ansprüche 46 bis 52, worin mindestens eine der Kammern (12, 13) eine steife Kammer mit mindestens eine Wand, die zum Gleiten in der Kammer vorgesehen ist, ist.

54. Einwegvorrichtung nach dem vorhergehenden Anspruch, worin mindestens eine Kammer (12, 13) eine Spritze ist.

55. Einwegvorrichtung nach dem vorhergehenden Anspruch, worin die Spritze eine verlängerbare Scheidewand umfasst, die blutundurchlässig ist und deren mindestens eine Abschnitt am Kolben befestigt ist und ein anderer Abschnitt an einem Kreisumfang des Spritzenzylinders befestigt ist.

56. Einwegvorrichtung nach einem der Ansprüche 48 bis zum vorhergehenden Anspruch, worin die zweite Kammer (13) eine Öffnung nach außen umfasst, umfassend eine hydrophobe Scheidenwand (41), die mit der benannten Öffnung assoziiert ist.

**57.** Einwegvorrichtung nach einem der Ansprüche 46 bis zum vorhergehenden Anspruch, worin mindestens ein Abschnitt der Kammer oder Kammern (12, 13) durchsichtig ist und eine Skala zum visuellen Lesen der Speicherfähigkeit umfasst.

**58.** Einwegvorrichtung nach einem der Ansprüche 46 bis zum vorhergehenden Anspruch, worin mindestens eine Kammer (12, 13) in die Behandlungseinheit (3) integriert ist.

**59.** Einwegvorrichtung nach einem der Ansprüche 46 bis zum vorhergehenden Anspruch, worin die beiden Speichermittel (12, 13) dieselbe Struktur und denselben maximalen Gehalt aufweisen.

**Revendications**

**1.** Dispositif pour le traitement extracorporel de sang (1) apte à fonctionner dans un mode à une seule aiguille (2), comprenant:

- une unité de traitement (3) ayant un premier compartiment (4) et un deuxième compartiment (5) séparés par une membrane semi-perméable (6),
- un circuit extracorporel de sang comprenant une seule aiguille (2), une ligne d'alimentation (7) en communication de fluide avec le premier compartiment (4) de l'unité de traitement (3), le premier compartiment (4), et une ligne de retour (8) en communication de fluide avec le premier compartiment (4) de l'unité de traitement (3),
- une ligne d'évacuation (9) à la sortie du deuxième compartiment (5),
- un moyen de fermeture (10, 11) agissant sur au moins la ligne d'alimentation (7) et la ligne de retour (8) afin de générer la séquence alternée d'alimentation et restitution de sang,
- au moins une première chambre (12) en communication de fluide avec le circuit extracorporel de sang et définissant un premier volume de sang additionnel, la première chambre (12) ayant un contenu total variable;
- la première chambre (12) est rigide et comprend au moins une paroi mobile,
- le dispositif comprend un moyen (19) agissant sur la chambre (12) afin de modifier le volume de la chambre (12), permettant ainsi de stocker le sang pendant la phase artérielle et de faire sortir le sang pendant la phase veineuse,

**caractérisé en ce que**:

la première chambre (12) comporte une ouverture vers l'extérieur, comprenant un diaphragme hydrophobe (41) associé à ladite ouverture et permettant d'éliminer les bulles d'air indésirables éventuellement présentes dans la chambre (12).

**2.** Dispositif selon la revendication 1, **caractérisé en ce que** la première chambre (12) est en communication de fluide avec la ligne d'alimentation (7).

**3.** Dispositif selon la revendication 1, **caractérisé en ce que** la première chambre (12) est en communication de fluide avec la ligne de retour (8).

**4.** Dispositif selon la revendication 1, comprenant une deuxième chambre (13) en communication de fluide avec le circuit extracorporel de sang et définissant un deuxième volume de sang additionnel, ladite deuxième chambre (13) étant rigide et comprenant au moins une paroi mobile; la deuxième chambre ayant un contenu total variable permettant de stocker le sang pendant la phase artérielle et de faire sortir le sang pendant la phase veineuse.

**5.** Dispositif selon la revendication 2 et la revendication précédente, où la deuxième chambre (13) est en communication de fluide avec la ligne de retour (8).

**6.** Dispositif selon une des revendications précédentes, comprenant un moyen de réglage du débit de fluide (14) agissant sur le circuit extracorporel de sang, où ledit moyen de réglage du débit de fluide comporte une pompe.

**7.** Dispositif selon la revendication précédente, où ledit moyen de réglage du débit de fluide (14) agit en amont du premier compartiment (4) de l'unité de traitement.

**8.** Dispositif selon la revendication précédente, où ledit moyen de réglage du débit de fluide (14) agit en amont de la première chambre (12).

**9.** Dispositif selon la revendication 7, où ledit moyen de réglage du débit de fluide (14) agit en aval de la première chambre (12).

**10.** Dispositif selon la revendication 6, où ledit moyen de réglage du débit de fluide (14) agit en aval du premier compartiment (4) de l'unité de traitement.

**11.** Dispositif selon la revendication 10, où ledit moyen de réglage du débit de fluide (14) agit en amont de la deuxième chambre (13).

**12.** Dispositif selon la revendication 10, où ledit moyen de réglage du débit de fluide (14) agit en aval de la deuxième chambre (13).

**13.** Dispositif selon une des revendications précédentes, comprenant au moins un détecteur d'air (17) placé en amont de la partie du moyen de fermeture veineuse (10, 11) agissant sur la ligne de retour (8).

**14.** Dispositif selon une des revendications précédentes, comprenant au moins un séparateur d'air (18).

**15.** Dispositif selon la revendication précédente, où le séparateur d'air est relié en aval de la chambre ou des chambres (12, 13) et de l'unité de traitement (3), et directement en amont du détecteur d'air (17).

**16.** Dispositif selon une des revendications 14 à 15, où le séparateur d'air (18) comporte un piège à bulles.

**17.** Dispositif selon une des revendications 14 et 15, où le séparateur d'air (18) est installé dans chaque chambre (12, 13).

**18.** Dispositif selon une des revendications précédentes, où au moins une des chambres (12, 13) est une chambre rigide avec au moins une paroi apte à glisser dans la chambre.

**19.** Dispositif selon la revendication précédente, où au moins une des chambres est une syringe.

**20.** Dispositif selon la revendication précédente, où au moins une syringe est positionnée verticalement avec la sortie de l'aiguille vers le haut.

**21.** Dispositif selon la revendication 19 ou 20, où la syringe comprend un diaphragme extensible et imperméable aux bactéries, dont au moins une partie est fixée au piston et une autre partie est fixée à une circonférence du cylindre de la syringe.

**22.** Dispositif selon une des revendications 4 à la précédente, où la deuxième chambre (13) comporte une ouverture vers l'extérieur, comprenant un diaphragme hydrophobe (41) associé à ladite ouverture et permettant d'éliminer les bulles d'air indésirables éventuellement présentes dans la deuxième chambre (13).

**23.** Dispositif selon une des revendications 19 à 22, où au moins une partie des chambres (12, 13) est transparente et comporte une échelle pour la lecture visuelle du contenu.

**24.** Dispositif selon une des revendications précédentes, où au moins une chambre (12, 13) est intégrée dans l'unité de traitement (3).

**25.** Dispositif selon une des revendications 19 à 24, où les deux chambres (12, 13) ont la même structure et le même contenu maximum.

**26.** Dispositif selon une des revendications précédentes, comprenant une unité de calcul et de commande (15) pour:

a. commander simultanément les moyens de fermeture (10, 11) afin d'alterner la phase artérielle et la phase veineuse, et
b. commander le remplissage d'au moins une des deux chambres (12, 13) pendant la phase artérielle et l'évacuation d'au moins une des deux chambres (12, 13) pendant la phase veineuse.

**27.** Dispositif selon une des revendications précédentes, où le moyen (19) agissant sur les chambres (12, 13) pour varier le volume des chambres (12, 13) comprend un moyen d'actionnement pour varier le volume de la chambre en déplaçant au moins une paroi de la chambre, dans ce cas-là ce moyen pouvant comporter un piston.

**28.** Dispositif selon la revendication précédente, où ledit moyen (19) comprend un moteur couplé au moyen d'actionnement.

**29.** Dispositif selon une des revendications 26 à 28, où l'unité de calcul et de commande (15) comprend des moyens pour commander le moyen (19) agissant sur les chambres (12, 13) pour varier le volume des chambres (12, 13) afin de remplir au moins une des deux chambres (12, 13) pendant la phase artérielle, et d'évacuer au moins une des deux chambres (12, 13) pendant la phase veineuse.

**30.** Dispositif selon une des revendications 26 à 29, où l'unité de calcul et de commande (15) comprend des moyens pour commander le premier moyen de réglage du débit de fluide (14) afin d'assurer un débit substantiellement constant directement en aval de celui-ci.

**31.** Dispositif selon une des revendications 26 à 30, où l'unité de calcul et de commande (15) comprend des moyens pour commander le moyen pour varier le volume des deux chambres (12, 13) afin d'établir un volume dans la première chambre (12) substantiellement égal au volume dans la deuxième chambre (13) pendant le fonctionnement du dispositif.

**32.** Dispositif selon une des revendications 26 à 31, comprenant:

> i. une interface utilisateur (16) pour recevoir les paramètres prévus;
> ii. des moyens de mesure pour établir les paramètres mesurés;
> iii. où le moyen de calcul et de commande (15) comprend des moyens pour calculer les paramètres de commande à partir d'un ou plusieurs paramètres prévus et d'un ou plusieurs paramètres mesurés, et pour commander le dispositif en fonction de ces paramètres.

**33.** Dispositif selon la revendication précédente, où les moyens de mesure comprennent des moyens de mesure de la pression ($P_A$, $P_V$) pour mesurer la pression artérielle et la pression veineuse.

**34.** Dispositif selon la revendication précédente, où les moyens de mesure comprennent au moins un débitmètre (D1, D2) pour établir le débit d'ultrafiltration.

**35.** Dispositif selon la revendication 33 ou 34, où les paramètres prévus comprennent au moins le volume systolique prévu $SV_{int}$.

**36.** Dispositif selon une des revendications 32 à 35, où les paramètres prévus comprennent le débit sanguin prévu $Q_{Bint}$ à l'entrée du premier compartiment (4).

**37.** Dispositif selon la revendication précédente, où les paramètres de commande comprennent au moins la durée de la phase artérielle $T_A$ et la durée de la phase veineuse $T_V$.

**38.** Dispositif selon la revendication 36, où les paramètres de commande comprennent au moins la vitesse d'actionnement du moyen d'actionnement $V_{ACT}$.

**39.** Dispositif selon la revendication 37 ou 38, où l'unité de calcul et de commande comprend des moyens pour régler le débit sanguin $Q_B$ autour du débit sanguin prévu $Q_{Bin}$, en fonction des paramètres mesurés $P_A$ et $P_V$.

**40.** Dispositif selon une des revendications 32 à 35, où les paramètres prévus comprennent la vitesse d'actionnement prévue $V_{ACTint}$ du moyen d'actionnement.

**41.** Dispositif selon une des revendications 32 à 35, où les paramètres prévus comprennent la durée prévue de la phase artérielle $T_{Aint}$ et la durée prévue de la phase veineuse $T_{Vint}$.

**42.** Dispositif selon une des revendications 40 et 41, où les paramètres de commande comprennent au moins le débit sanguin $Q_B$.

**43.** Dispositif selon la revendication 42, où l'unité de calcul et de commande comprend des moyens pour régler la vitesse d'actionnement $V_{ACT}$ autour de la vitesse d'actionnement prévue $V_{ACTint}$ en fonction des paramètres mesurés $P_A$ et $P_V$.

**44.** Dispositif selon la revendication 42, où l'unité de calcul et de commande comprend des moyens pour régler la durée de la phase artérielle $T_A$ autour de la durée prévue pour la phase artérielle $T_{Aint}$ et pour régler la durée de la phase veineuse $T_V$ autour de la durée prévue pour la phase veineuse $T_{Vint}$ en fonction des paramètres mesurés $P_A$ et $P_V$.

**45.** Dispositif selon une des revendications 26 à 34, où l'unité de calcul et de commande comprend des moyens pour commander les modes de fonctionnement suivants: hémodialyse, hémofiltration, hémodiafiltration.

**46.** Dispositif jetable (100) pour l'emploi dans un circuit extracorporel de sang (1) avec une seule aiguille (2), comprenant:

a. une unité de traitement (3) ayant un premier compartiment (4) et un deuxième compartiment (5) séparés par une membrane semi-perméable (6);
b. un circuit extracorporel de sang comportant une seule aiguille (2), une ligne d'alimentation (7), le premier compartiment (4) de l'unité de traitement (3) et une ligne de retour (8);
c. au moins une première chambre (12) pour définir un volume de sang additionnel, comportant un raccordement (22) en communication de fluide avec la ligne d'alimentation (7) ou avec la ligne de retour (8),
d. la première chambre (12) ayant un contenu total variable,
e. la première chambre (12) étant rigide et comportant au moins une paroi mobile, **caractérisé en ce que**
f. la première chambre (12) comporte une ouverture vers l'extérieur, comprenant un diaphragme hydrophobe (41) associé à ladite ouverture.

**47.** Dispositif jetable selon la revendication précédente, où la première chambre (12) est en communication de fluide avec la ligne d'alimentation (7) au moyen dudit raccordement (22), qui est fixe ou amovible.

**48.** Dispositif jetable selon la revendication précédente, comprenant une deuxième chambre (13) pour définir un deuxième volume de sang additionnel, qui comporte un deuxième raccordement fixe ou amovible (23) pour mettre la deuxième chambre (13) en communication de fluide avec la ligne de retour (8), la deuxième chambre (13) ayant un contenu total variable, la deuxième chambre (13) étant rigide et comportant au moins une paroi mobile.

**49.** Dispositif jetable selon une des revendications 46 à 48, comprenant au moins une partie (14b) étant à même de coopérer avec un moyen de réglage du débit (14).

**50.** Dispositif jetable selon la revendication précédente, où ladite partie (14b) est placée sur la ligne d'alimentation (7) en aval du premier raccordement (22).

**51.** Dispositif jetable selon une des revendications 46 à 50, comprenant une partie (17b) placée sur la ligne de retour (8) et étant à même de coopérer avec un détecteur d'air (17).

**52.** Dispositif jetable selon une des revendications 46 à 50, comprenant au moins un séparateur d'air (18) branché sur la ligne de retour (8).

**53.** Dispositif jetable selon une des revendications 46 à 52, où au moins une des chambres (12, 13) est une chambre rigide avec au moins une paroi apte à glisser dans la chambre.

**54.** Dispositif jetable selon la revendication précédente, où au moins une chambre (12, 13) est une syringe.

**55.** Dispositif jetable selon la revendication précédente, où la syringe comporte un diaphragme extensible imperméable au sang, dont au moins une partie est fixée au piston et une autre partie est fixée à une circonférence du cylindre de la syringe.

**56.** Dispositif jetable selon une des revendications 46 à la revendication précédente, où la deuxième chambre (13) comporte une ouverture vers l'extérieur, comprenant un diaphragme hydrophobe (41) associé à ladite ouverture.

**57.** Dispositif jetable selon une des revendications 46 à la revendication précédente, où au moins une partie de la chambre ou des chambres (12, 13) est transparente et comprend une échelle pour la lecture visuelle de la capacité de stockage.

58. Dispositif jetable selon une des revendications 46 à la revendication précédente, où au moins une chambre (12, 13) est intégrée dans l'unité de traitement (3).

59. Dispositif jetable selon une des revendications 46 à la revendication précédente, où les deux moyens de stockage (12, 13) ont la même structure et le même contenu maximum.

FIG 1

STATE OF THE ART

FIG 2

STATE OF THE ART

STATE OF THE ART

# FIG 3

EP 1 722 837 B1

FIG 4

STATE OF THE ART

FIG 5

STATE OF THE ART

FIG 6

STATE OF THE ART

Invention — 1st embodiment

FIG 7

Invention — 1st embodiment

FIG 8

FIG 9

Invention — 2nd embodiment

FIG 10

Invention — 3rd embodiment

Invention – 4th embodiment

FIG 11

FIG 13 arterial phase

Invention — 4th embodiment

Invention — 4th embodiment

FIG 14 venous phase

FIG 15

**EP 1 722 837 B1**

**Patent documents cited in the description**

- FR 2584906
- FR 2672217
- EP 0392304 A
- EP 0462422 A
- FR 2548906